# EUROPEAN PATENT SPECIFICATION

(11) **EP 1 747 789 B1**
(45) Date of publication and mention of the grant of the patent: **19.08.2009**
(21) Application number: 06121820.2
(22) Date of filing: 30.06.2003
(51) Int. Cl.: A61M 5/34

(54) **A needle mount and use of the needle mount for mounting a needle assembly**
Nadelhalter und Verwendung des Nadelhalters zur Montage einer Nadelanordnung
Dispositif de montage d'aiguille et utilisation du dispositif pour monter un ensemble aiguille

(30) Priority: 03.07.2002 US 394083 P; 01.08.2002 DK 200201169
(43) Date of publication of application: 31.01.2007
(62) Divisional of application: 03762466.5
(73) Proprietor: NOVO NORDISK A/S, 2880 Bagsvaerd (DK)
(72) Inventor: Klint, Hendrik Soenderskov, 2800 Lyngby (DK); Radmer, Jim, 3480 Fredensborg (DK); Smedegaard, Jorgen K., 2720 Vanlose (DK); Nielsen, Jan Frank, 2800 Lyngby (DK); Moeller Jensen, Peter, 2970 Hoersholm (DK); Moeller Jensen, Jens, 1051 Copenhagen K (DK)

(56) References cited:
- EP-A2- 0 055 859
- WO-A-02/11798
- WO-A-96/11028
- CH-A- 332 340
- GB-A- 594 366
- US-A- 1 757 680

## Description

### THE TECHNICAL FIELD OF THE INVENTION

The present invention relates generally to injection devices and, in particular, provides needle mounts for mounting a needle to an injection device or to an ampoule that my be mounted in the injection device.

### DESCRIPTION OF RELATED ART

injection devices, also referred to as dosers, have greatly improved the lives of patients who must self-adminster drugs and biological agents. Dosers may take many forms, including simple disposable devices that are little more than an ampoule with an injection means or they may be highly sophisticated instruments with numerous functions. Regardless of their form, they have proven to be great aids in assisting patients to self-adminster injectable drugs and biological agents. They also greatly assist care givers in administering injectable medicines to those incapable of performing self-injections.

In particular, pen-style injection devices, have proven to be an accurate, convenient, and often discrete, way to administer drugs and biological agents, such as insulin. Modern devices have become more sophisticated and often include diverse and robust functions, such as memories for remembering time and amount of last dose, as well as, in the case of insulin devices, blood glucose monitors. While pen-style dosers are typically cylindrically shaped with needles protruding from the most distal portion of one end of the device, some of the more modern and /or sophisticated dosers have other shapes with the needle no longer protruding from the most distal part of an end of the device. (See e.g., Innovo® and InnoLet® from Novo Nordisk A/S Bagsvaerd Denmark).

Typically, injection devices use a pre-filled cartridge containing the medication of interest. The cartridge may be an integral part of the doser or it may comprise an ampoule having a membrane at one. See U.S. Patent No. 6,312,413 to Jensen et. al, which is hereby incorporated by reference. Often the end of the ampoule having the membrane is fitted with a needle mount. The needle mount usually comprises a threaded mounting surface to allow a needle assembly, such as a needle and hub assembly, to be screwed on. The needle mount may be an integral part of the ampoule or may be a separate adapter top (see U.S. Patent Nos. 5,693,027 and 6,126,646, which are hereby incorporated by reference) that is mounted to the ampoule. Of course, some dosers have needle mounts that are integral parts of the doser.

The prior art document CH 332.340 discloses a needle mounting system for mounting a needle assembly onto a needle mount of an injection device. The needle assembly comprises a needle attached to a hub, which hub has an interior threaded wall part. The needle mount shows the combination of features of the preamble of claim 1. It comprises a cylindrical outer wall having a top end and threads disposed on the cylindrical outer wall. Further two grooves are disposed in the cylindrical outer wall beginning at the top end of the cylindrical wall and defining a passageway that is generally parallel to a cylindrical axis of the cylindrical outer wall. The interior threaded wall parts of the hub travel in these two grooves during mounting of the needle assembly on the needle mount.

A further prior art document US 5.019.045 discloses a needle assembly comprising a hub and a needle attached to the distal end of the hub.

In the typical injection device where the needle mount is not part of the doser, the end of the ampoule having the needle mount protrudes from the injection device. Where the needle mount is part of the doser, the needle mount is usually disposed on an outer end of the doser. In either embodiment, the needle hub is then screwed onto the needle mount. One disadvantage of the prior art needle mounting systems is that they require the patient to screw the needle hub onto the end of the ampoule, or the doser, by turning the needle relative to the device several times. For patients with dexterity problems, this is inconvenient. Moreover, it is often desirable to store needles for the injection devices in a magazine. Often many newer generation injection devices are not cylindrical and in many new devices, other parts of the device extend past the needle mount making it impossible to mount the needle on the injection device without first removing it from the magazine.

### SUMMARY OF THE INVENTION

The present invention provides a needle mount as defined in claim 1. The present invention allows a needle and hub assembly to be mounted on an ampoule and/or injection device without having to rotate completely the needle hub assembly relative to the injection device. The needle assembly also includes a means for mounting the hub to a needle mount with only a partial rotation of the needle hub relative to the mount.

At least one embodiment of the present invention includes the use of a needle assembly in combination with a needle mount according to claim 1. In this embodiment, a plurality of protrusions extends radially inward from the wall of the hub. Typically, the hub wall is cylindrical. The needle mount according to the present invention, includes a structure having a cylindrical outer wall. A plurality of grooves is disposed on the outer wall. The grooves begin at the top of the wall and contain at least two portions: a first portion that defines a passageway that is substantially parallel to the cylindrical axis of the outer wall, and a second portion that is oriented at an angle to the first portion.

The present invention therefore provides for mounting needles to injection devices. It may be useful in mounting needles stored in magazines and is particularly useful for injection devices that have a portion that extends past the needle mount. The injection device can be partially inserted into a magazine holding needle assemblies, and may be rotated relative to the magazine by less than a full revolution and may be then removed with the needle assembly attached thereto.

The needle assembly includes a cylindrical hub that has a needle mounted thereon.

### BRIEF DESCRIPTION OF THE DRAWINGS

Figure 1 is a three-dimensional view of a needle hub and a needle mount according to the present invention.
Figure 2 is a cut-away view of the needle mount and needle hub shown in Figure 1.
Figure 3 is a three-dimensional view of a needle assembly and a needle mount which is not according to the present invention.
Figure 4 illustrates the embodiment of Figure 3 when viewed from below.
Figure 5 is a cut-way view of the needle assembly of Figures 3-4.
Figure 6 is an enlarged view of the needle assembly mounting means of the device shown in Figures 3-5.
Figure 7 is a cut through view of a needle mount and needle hub for tacitly determining whether the needle hub is securely mounted on the needle mount.
Figure 8 is a side view of a magazine for storing needles that may be used in combination with the present invention.
Figure 9 is a top view of the magazine shown in Figure 8.

### DETAILED DESCRIPTION OF THE INVENTION

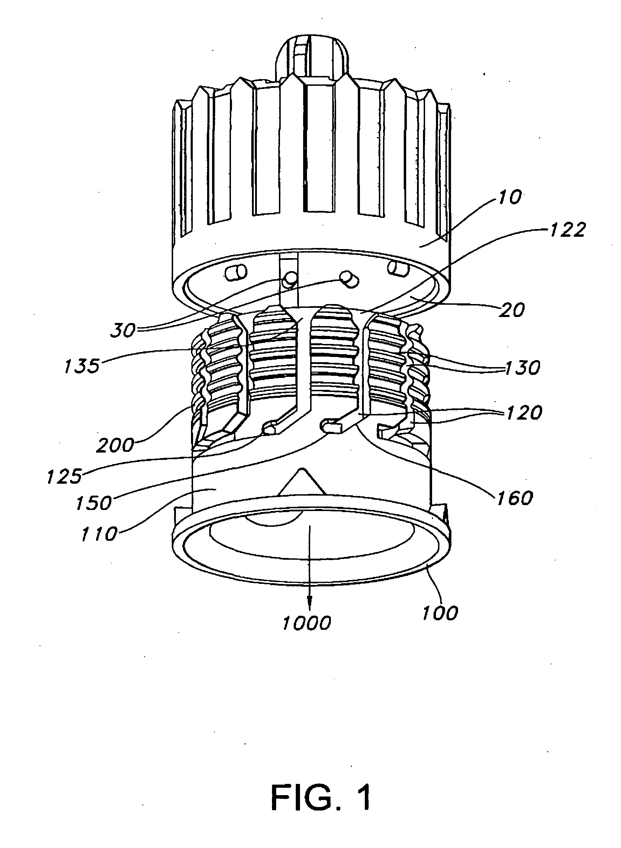

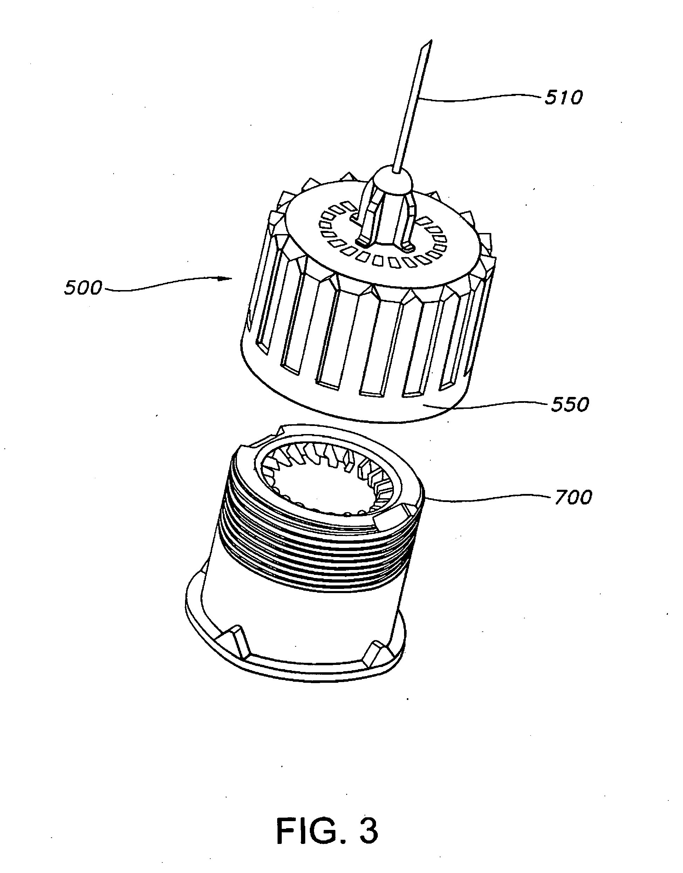

The present invention provides a needle mount (100) for attaching needle hub assemblies to ampoules and injection devices. Typically, a needle hub assembly comprises a needle **510** mounted to a hub **500** (see e.g. Figure 3). As is shown in Figure 1, a needle hub **10** may be generally cylindrically shaped and have an interior wall surface **20.** In one embodiment of the present invention, a plurality of protrusions **30** extends radially inward from the interior surface 20.

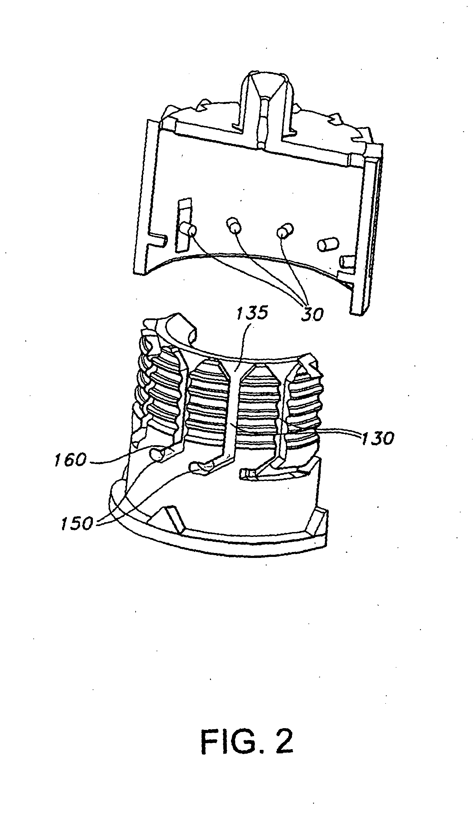

The needle mount **100** is designed to accept the needle hub **10.** (See e.g. Fig. 1). As is shown in Figures 1 and 2, the needle mount **100** may be generally cylindrically shaped and have an exterior wall surface **110.** A plurality of grooves or slots **120** are disposed in the exterior surface **110.** The grooves **120** have a first end **122** and a second end **125.** The grooves **120** have a first portion **130** that defines a passageway that is generally parallel to the cylindrical axis **1000** of the needle mount **100.** While the first portion of the groove **130** is shown in the drawings as having a rectangular portion, the exact shape of the groove is not critical so long as it allows the protrusions **30** on the needle hub to move in a direction parallel to the cylindrical axis 1000. Thus, while the groove may have walls that are not necessarily parallel to the cylindrical axis **1000,** the groove may still be said to be parallel to the cylindrical axis if it allows the protrusions **30** to move in a direction parallel to the cylindrical axis. The first portion of the grooves **130** may have width that is wider than the remainder of the first portion or the remainder of the groove **130.** In embodiments where the groove has walls that are not parallel to the cylindrical axis **1000,** the width of the first portion of the groove **130** may be the average width for the first portion of the groove **130.**

The first portion **130** may have an entrance **135** that has a width dimension that is greater than the average width of the first portion or is wider than the average width of the entire groove **120.** The entrance **135** may act as an alignment means for aligning the needle hub so that the protrusions will enter the groove **120.** In most embodiments, but not all, the entrance width is wider than any other point in the groove **120.** Typically the width of the groove narrows as the groove is traversed away from the entrance **135.** As is shown, the groove may reach a constant width at some distance from the opening. In some embodiments the width of the first portion **130** is widest at the entrance **135** and continues to narrow over the length of the first portion **130.** The grooves also have a second portion **150** that is either perpendicular to the cylindrical axis **1000,** or lies at angle to the first portion **130.** In some embodiments of the present invention the second portion **150** may be comprised of only one surface that is generally perpendicular to the cylindrical axis of the needle mount. Thus, the second portion of the groove **150** need not be a slot having two sides, but needs only one side to prevent protrusions on the needle hub from moving toward the outer end of the needle mount. As shown in Figure 1, the grooves **120** may also have a third portion **160** that is oriented at an angle to the first portion **130** and the second portion **150.**

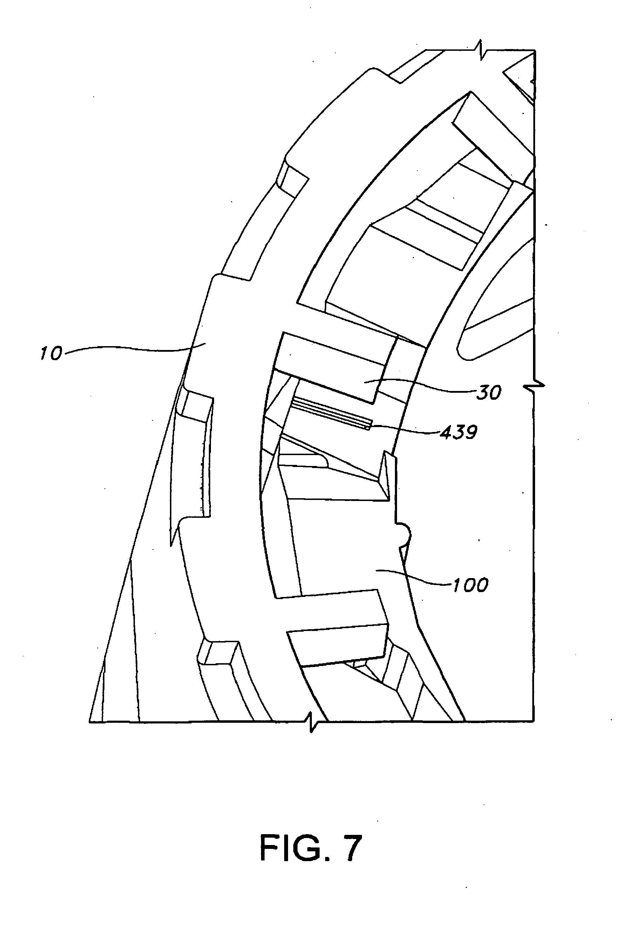

A means for tacitly determining whether the needle assembly is securely fixed to the hub may be provided. This may be accomplished in numerous different ways, including providing a small projection(s) **439** at the side or in the bottom of the second portion of the grooves **120.** (See e.g. Figure 7). The protrusions 30 have to overcome the projections **439** before the needle is fixed. The deformation of the projections may cause a tacitly feel or a sound, such as a clicking sound. Thus, the needle mounting system can be designed so that the needle hub and the needle mount generate a clicking sound when the needle is securely placed on the mount. When the hub is to be remounted from injection device the oblique tactile protrusions can be more sharp at their ends, so that hub is better fixed during injection and handling etc. This also makes it possible for the patient to keep the needle for more injection.

One advantage of the present invention is that the needle mount, may be equipped with standard threads **200** on its exterior surface. (See Fig. 1). The grooves **120** may be cut into the standard threads **200.** This allows the needle mount **100** of the present invention to accept not only needle hubs , but also standard, threaded needle-hub assemblies.

While Figure 1 shows the grooves on the needle mount and the protrusions on the needle hub, the needle mount may be configured with the grooves located on the interior surface of the needle hub and the protrusions extending outward from the exterior wall of the needle mount. It may be advantageous to size and shape the protrusions so that they fit between standard threads used with existing needle hubs. The protrusions may then be arranged on the exterior wall of the needle mount to allow not only needle hub assemblies having grooves in their interior wall to be attached, but also standard, threaded needle hubs.

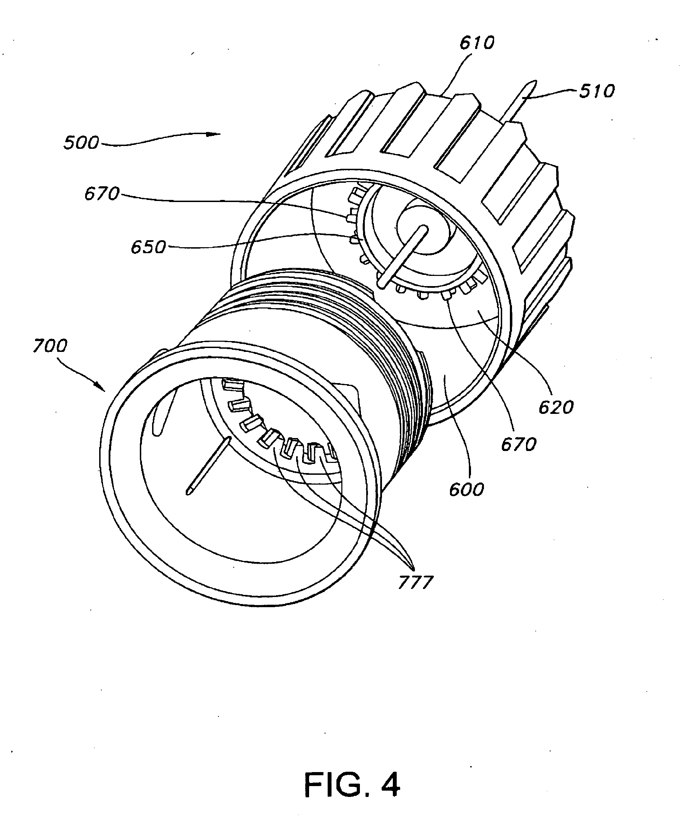

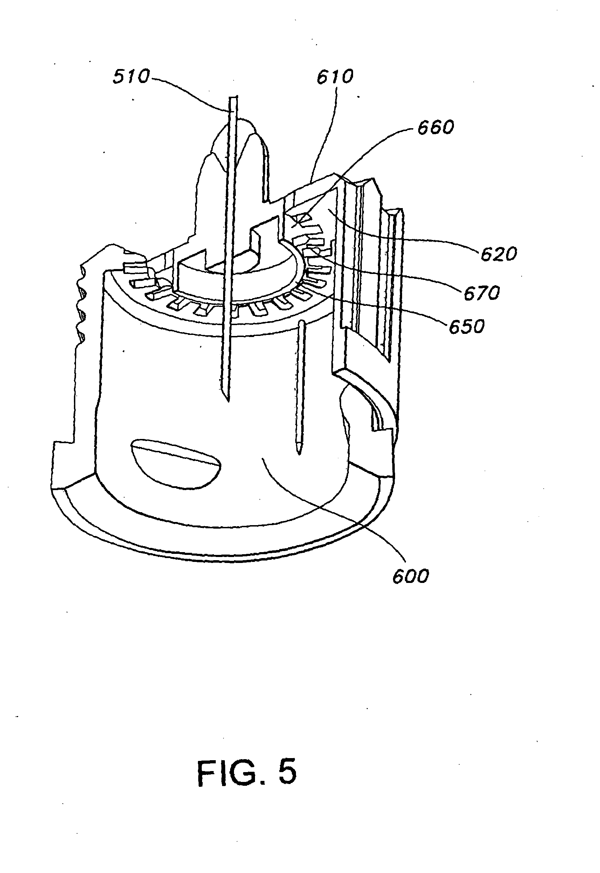

As is shown in Figures 3-6, which do not show embodiments according to the invention, the needle hub assembly **500** has a needle **510** mounted thereto. The needle hub **550** may be generally cylindrically shaped and has an interior wall surface **600** and a closed top end **610.** The closed top end **610** has an inside surface **620.** A cylindrical member **650** protrudes from the inside surface **620** and has an outer surface **660.** See Fig. 5. Protrusions **670** extend radially outward from the outer surface **660.** The protrusions may take various forms and shapes, including the triangular prism shape shown in the drawings.

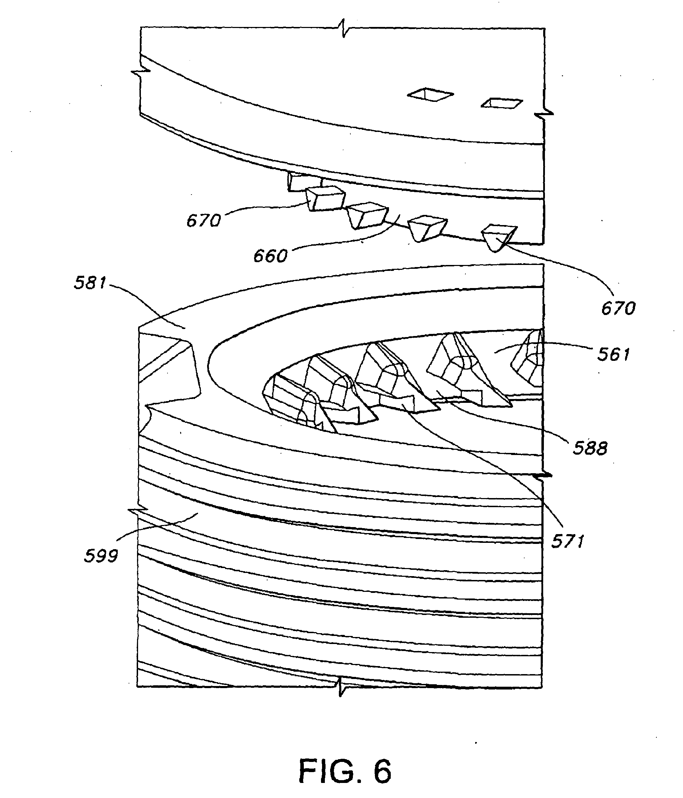

The needle hub assembly shown in Figures 3-5 may be used with a modified needle mount, **700.** As is shown in Figures 3 - 6, the needle mount **700** may be generally cylindrically shaped and have a top end, an interior surface, an exterior surface, and a plurality of locking elements (which may be additional protrusions) extending from the interior surface inward. The locking elements may be arranged to form passageways for the protrusions **500** on the needle mount, thereby forming a plurality of grooves for accepting the protrusions from the needle hub assembly **500.** As is shown in Fig. 6, the grooves may have a first portion **561** that defines a passageway that is generally parallel to the cylindrical axis of the needle mount, a second portion **571** that is perpendicular to the cylindrical axis and a third portion **588** that connects the second **571** and first portions **561.** The first portion **561** may be widest at its opening and thus act as an alignment mechanism for the protrusions on the needle hub. The needle mount may have a mounting surface **581** on which a portion of the needle hub rests when the needle hub is mounted on the needle mount. The mounting surface may be a top edge of the top end of needle mount, or it may be the exterior wall surface **599** of the needle mount or both. The embodiment shown in Figures 2-4 also advantageously allows the outer surface of the needle mount to have threads so that standard prior-art needle hubs may be used with the improved needle mount of the present invention.

The present invention enables various methods for attaching a needle-hub assembly to an ampoule or injection device. For example, a needle mount can be inserted into a needle hub, the needle hub can be rotate relative to the needle mount less than one revolution - typically between 5 and 30 to 60 degrees. A clicking noise or vibration or other tactile feedback will be provided to indicate that the needle is securely mounted to the hub. In some embodiments little rotation is necessary. In some embodiments, it is possible that no rotation is needed. The surface of the locking element **777** could simply force the hub to rotate upon insertion of the mount into the interior of the hub **500.** In other embodiments, more rotation may be required.

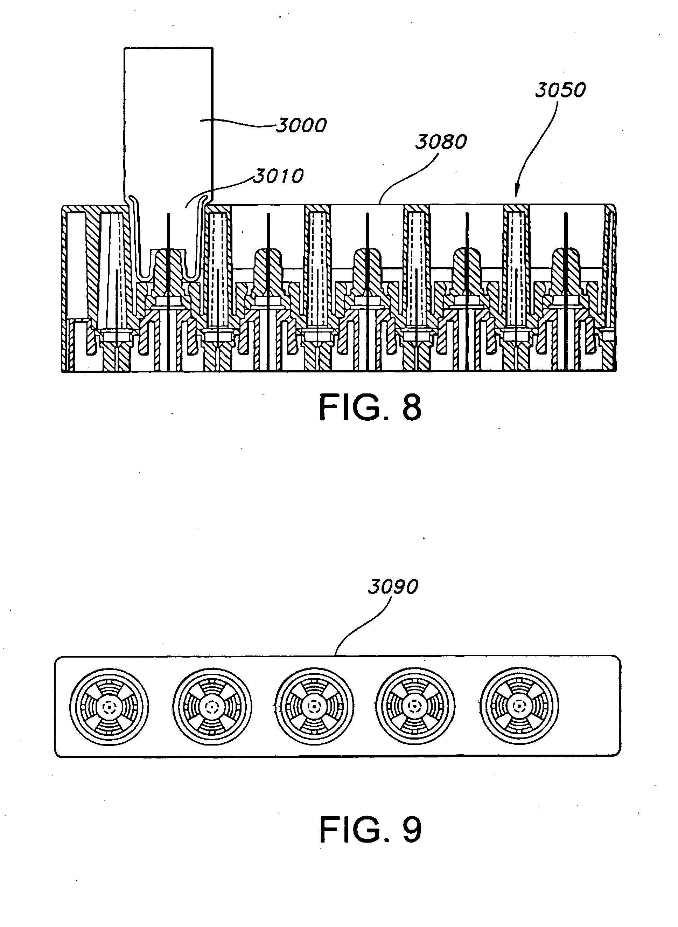

Because the mounting of a needle hub to a needle mount does not require that the hub be rotated a full revolution relative to the mount (i.e. either the hub is rotated and the mount is held stationary or the mount is rotated and the hub is held stationary, or both are turned in opposite direction), the present invention enables and provides for mounting of needle-hub assemblies stored in magazines, similar to that shown in Figures 8 and 9, to injection devices where their shape would not allow the device to be rotated relative to the magazine by a full revolution. In one embodiment, a portion of an injection device **3000,** usually the portion containing a needle mount **3010,** is inserted into a needle magazine **3050.** The device **3000,** without being rotated a full revolution is then removed with a needle fully attached to it. In some embodiments audible or tactile feedback is provided to indicate that the need is securely mounted to the device. In some embodiments, the portion of the device that is inserted into the magazine may be an end portion of an ampoule that extends from the device. Some methods of using the present invention may be performed using the needles that are stored in a magazine having a flush surface 3070 and the needle and hub assemblies **3080** are located below the surface **3070,** usually - but not necessarily - in recessed cavities **3090** (see Figure 9).

The foregoing is a brief description of some exemplary embodiments of the present invention and is intended to be illustrative and not exhaustive of the present invention. Those of skill in the art will recognize the nature of language makes it impossible to capture the essence of all aspects of the present invention and unimportant and insubstantial substitutes for various elements are intended to be included within the scope of the invention as defined by the following claims.

## Claims

1. A needle mount (100) comprising;
A cylindrical outer wall (110) having a top end and threads (200) disposed on the cylindrical outer wall (110) for accepting thereon a standard threaded needle assembly, and a plurality of grooves (120) disposed in the cylindrical outer wall (110) beginning at the end of the cylindrical outer wall (11) and defining a passageway that is generally parallel to a cylindrical axis (1000) of the cylindrical outer wall (110),
**Characterized in that**,
at least one groove (120) of the needle mount (100) further comprises a first portion (130) and a second portion (150) oriented at an angle to the first portion (130).

2. A needle mount (100) according to claim 1 **characterized in that**, the angle between the first portion (130) and the second portion (150) is 90 degrees or less.

3. A needle mount (100) according to claim 1 or 2, **characterized in that** the groove (120) is widest at the top end of the needle mount (100) forming an entrance (135).

4. Use of a needle assembly (500) for mounting the assembly on a needle mount, the assembly comprising a hub (10) attached to a needle (510), the hub (10) having an interior cylindrical wall (20) with a plurality of protrusions (30) extending radially inward from the interior cylindrical wall (20) the use comprising providing needle mount according to any of the claims 1 to 3, and mounting the needle assembly on said needle mount.

5. Use of a needle assembly (500) according to claim 4, **characterized in that**, the protrusions (30) comprises a circular surface.

6. Use of a needle assembly (500) according to claim 4 or 5, **characterized in that**, the protrusions (30) extends perpendicular to a cylindrical axis (1000)

7. Use of a needle assembly (500) according to anyone of the claims 4 to 6, **characterized In that** the protrusions (30) are located in the proximity of a proximal end of the hub (10) opposite the top end.

## Patentansprüche

1. Nadelbefestigung (100), umfassend;
eine zylinderförmige Außenwand (110) mit einem oberen Ende und Gewinden (200), die an der zylinderförmigen Außenwand (110) angeordnet sind, um daran eine mit Standardgewinde versehene Nadelanordnung aufzunehmen, und eine Mehrzahl von Rillen (120), die in der zylinderförmigen Außenwand (110) derart angeordnet sind, dass sie am oberen Ende der zylinderförmigen Außenwand (11) beginnen und einen Durchgangsweg definieren, der im Allgemeinen parallel zu einer Zylinderachse (1000) der zylinderförmigen Außenwand liegt,
**dadurch gekennzeichnet, dass**
mindestens eine Rille (120) der Nadelbefestigung (100) des Weiteren einen ersten Teilbereich (130) und einen zweiten Teilbereich (150), der mit einem Winkel zum ersten Teilbereich (130) orientiert ist, umfasst.

2. Nadelbefestigung (100) nach Anspruch 1, **dadurch gekennzeichnet, dass** der Winkel zwischen dem ersten Teilbereich (130) und dem zweiten Teilbereich (150) 90 Grad oder weniger beträgt.

3. Nadelbefestigung (100) nach Anspruch 1 oder 2, **dadurch gekennzeichnet, dass** die Rille (120) am oberen Ende der Nadelbefestigung (100) am breitesten ist, wodurch ein Zugang (135) definiert wird.

4. Verwendung einer Nadelanordnung (500) zum Befestigen der Anordnung an einer Nadelbefestigung, wobei die Anordnung einen an einer Nadel (510) angebrachten Sitz (10) umfasst, wobei der Sitz (10) eine zylinderförmige Wand (20) mit einer Vielzahl von sich von der zylinderförmigen Innenwand (20) radial nach innen erstreckenden Vorsprüngen (30) umfasst, wobei die Verwendung das Bereitstellen einer Nadelbefestigung nach einem der Ansprüche 1 bis 3 und Befestigen der Nadelanordnung an der Nadelbefestigung umfasst.

5. Verwendung einer Nadelanordnung (500) nach Anspruch 4, **dadurch gekennzeichnet, dass** die Vorsprünge (30) eine kreisförmige Oberfläche umfassen.

6. Verwendung einer Nadelanordnung (500) nach Anspruch 4 oder 5, **dadurch gekennzeichnet, dass** sich die Vorsprünge (30) senkrecht zu einer Zylinderachse (1000) erstrecken.

7. Verwendung einer Nadelanordnung (500) nach einem der Ansprüche 4 bis 6, **dadurch gekennzeichnet, dass** sich die Vorsprünge (30) in der Nähe eines Proximalendes des Sitzes (10) gegenüber des oberen Endes befinden.

## Revendications

1. Une monture d'aiguille (100) comprenant :
une paroi extérieure cylindrique (110) ayant une extrémité supérieure et des filets (200) disposés sur la paroi extérieure cylindrique (110) pour recevoir dessus un bloc d'aiguille taraudé standard, et une pluralité de gorges (120) disposées dans la paroi extérieure cylindrique (110) commençant à l'extrémité de la paroi extérieure cylindrique (11) et définissant un passage qui est généralement parallèle à un axe cylindrique (1000) de la paroi extérieure cylindrique (110),
**caractérisée en ce que**
au moins une gorge (110) de la monture d'aiguille (100) comprend en outre une première partie (130) et une seconde partie (150) orientée en formant un angle avec la première partie (130).

2. Une monture d'aiguille (100) selon la revendication 1, **caractérisée en ce que** l'angle entre la première partie (130) et la seconde partie (150) est de 90° ou moins.

3. Une monture d'aiguille (100) selon la revendication 1 ou 2, **caractérisée en ce que** la gorge (101) est plus large à l'extrémité supérieure de la monture d'aiguille (100) formant une entrée (135).

4. Utilisation d'un bloc d'aiguille (500) pour monter le bloc sur une monture d'aiguille, le bloc comprenant un pavillon (10) solidarisé à une aiguille (510), le pavillon (10) ayant une paroi cylindrique intérieure (20) avec une pluralité de saillies (30) s'étendant radialement vers l'intérieur depuis la paroi cylindrique intérieure (20), l'utilisation comprenant la mise à disposition d'une monture d'aiguille selon l'une des revendications 1 à 3 et le montage du bloc d'aiguille sur ladite monture d'aiguille.

5. Utilisation d'un bloc d'aiguille (500) selon la revendication 4, **caractérisé en ce que** les saillies (30) comprennent une surface circulaire.

6. Utilisation d'un bloc d'aiguille (500) selon la revendication 4 ou 5, **caractérisé en ce que** les saillies (30) s'étendent perpendiculairement à un axe de cylindre (1000).

7. Utilisation d'un bloc d'aiguille (500) selon l'une des revendications 4 à 6, **caractérisée en ce que** les saillies (30) sont situées à proximité d'une extrémité proximale du pavillon (10) à l'opposé de l'extrémité supérieure.
